# EUROPEAN PATENT APPLICATION

(11) **EP 3 626 728 A1**
(43) Date of publication of application: **25.03.2020**
(21) Application number: 18845981.2
(22) Date of filing: 19.06.2018
(51) Int. Cl.: C07K 7/08, A23L 33/18, A61K 38/00

(54) **PEPTIDE FOR INHIBITING BONE RESORPTION**

(30) Priority: 18.08.2017 KR 20170104939
(71) Applicant: Seoul National University R & DB Foundation, Seoul 08826 (KR); Nibec Co., Ltd., Chungcheongbuk-do 27816 (KR)
(72) Inventor: HAN, Seung Hyun, Seoul 06216 (KR); PARK, Ok-Jin, Seoul 06684 (KR); KIM, Jiseon, Seoul 08655 (KR); AHN, Ki Bum, Gwacheon-si Gyeonggi-do 13834 (KR); PARK, Yoon Jeong, Seoul 08291 (KR); CHUNG, Chong-Pyoung, Seoul 05618 (KR); LEE, Jue-Yeon, Gwacheon-si Gyeonggi-do 13831 (KR)
(74) Representative: Mewburn Ellis LLP
(86) International application number: PCT/KR2018/006874
(87) International publication number: WO 2019/035545

(57) **Abstract**

The present invention relates to a peptide for inhibiting bone resorption and, more specifically, to a peptide for inhibiting bone destruction by inhibiting the differentiation and activity of osteoclasts. According to the present invention, a peptide for inhibiting bone resorption can inhibit bone destruction by inhibiting the differentiation and activity of osteoclasts, and thus can be used as a therapeutic agent for various bone diseases caused by an excessive increase in the activity of osteoclasts.

<110>     SEOUL NATIONAL UNIVERSITY R AND DB FOUNDATION
          NANO INTELLIGENT BIOMEDICAL ENGINEERING CORPORATION. CO. LTD

<120>     Peptide for Inhibiting Bone Resorption

<130>     PP-B2038

<150>     KR 10-2017-0104939
<151>     2017-08-18

<160>     7

<170>     KoPatentIn 3.0

<210>     1
<211>     22
<212>     DNA
<213>     Artificial Sequence

<220>
<223>     c-Fos-F


<400>     1
gcccgactac gaggcgtcat cc                                                22


<210>     2
<211>     19
<212>     DNA
<213>     Artificial Sequence

<220>
<223>     c-Fos-R


<400>     2
tccggcactt ggctgcagc                                                    19


<210>     3
<211>     20
<212>     DNA
<213>     Artificial Sequence

<220>
<223>     NFATc1-F


<400>     3
tcggatcgag gtgcagccca                                                   20


<210>     4
<211>     20
<212>     DNA
<213>     Artificial Sequence

<220>
<223>     NFATc1-R


<400>     4

gggctgaccg ctgggaacac                                                    20

<210>    5
<211>    20
<212>    DNA
<213>    Artificial Sequence

<220>
<223>    b-actin-F

<400>    5
   gtggggcgcc ccaggcacca                                                     20

<210>    6
<211>    24
<212>    DNA
<213>    Artificial Sequence

<220>
<223>    b-actin-R

<400>    6
ctccttaatc tcacgcacga tttc                                               24

<210>    7
<211>    15
<212>    PRT
<213>    Artificial Sequence

<220>
<223>    HBD3-C15

<400>    7
Gly Lys Cys Ser Thr Arg Gly Arg Lys Cys Cys Arg Arg Lys Lys
1               5                   10                  15

## Description

### [Technical Field]

The present invention relates to a peptide for inhibiting bone resorption, and more specifically to a peptide for inhibiting bone destruction by inhibiting the differentiation and activity of osteoclasts.

### [Background Art]

Bone homeostasis is maintained through the balance between bone resorption and bone formation, and bones function to support the human body and protect internal organs. Resorption and formation of bones are regulated by bone-constituting cells called "osteoclasts" and "osteoblasts".

Osteoblasts secrete receptor activator of nuclear factor-kappaB ligand (RANKL) and a cytokine called osteoprotegerin (OPG). RANKL is expressed in osteoclast progenitor cells and binds to RANK to cause osteoclasts to be differentiated. When the osteoclasts are differentiated into mature osteoclasts, they induce bone resorption, and OPG is a decoy receptor that inhibits the formation of osteoclasts through binding to RANKL (Theill L. E. et al., Annu. Rev. Immunol. 20:795-823, 2002; Wagner E. F. et al., Curr. Opin. Genet. Dev. 11:527-532, 2001) .

Osteoporosis is a metabolic disease that occurs when osteoclasts are more active than osteoblasts due to the imbalance of bone re-formation resulting from excessively increased activity of osteoclasts (Iqbal MM et al., South Med J. 93(1):2-18, 2000), and leads to increased risk of fracture of the spine, thighs and radius due to decreased bone strength. Osteoporosis is known to be caused by aging, smoking, menopause, lack of exercise and the like, in particular, and is recognized as a serious health problem due to the prolonged life expectancy in accordance with advances in medicine.

Currently used osteoporosis therapeutics are produced based on the mechanism of inhibiting bone resorption, and 90% or more of osteoporosis therapeutics are bisphosphonate agents that inhibit bone resorption and are known to have an excellent effect of suppressing an osteoporotic fracture rate. However, side effects such as jawbone necrosis have been reported due to excessive inhibition of bone resorption upon long-term use (Coleman RE et al., Br J Cancer, 98:1736-1740, 2008). Therefore, there is a need for a new therapeutic agent for osteoporosis that is relatively economical and has no side effects.

Meanwhile, human beta defensin-3 is a human-derived antibiotic that is expressed in a large amount in the oral cavity, respiratory organs and digestive organs, and is known to have an antibiotic effect on bacteria (J Harder et al., J Biol Chem. 276(8):5707-13, 2001). Interestingly, it is known that positively charged amino acids play a key role in the antibiotic effect of human beta defensin-3, and a large amount of positively charged amino acids are present at the C-terminus of human beta defensin-3.

Recently, research on bone-related diseases has increased due to the acceleration of an aging population, and international attention has been focused on the development of drugs for the prevention and treatment of bone-related diseases.

Therefore, as a result of extensive effort to a discover a novel substance that is human-friendly and can be mass-produced and thereby develop a therapeutic agent for bone-related diseases without causing side effects, the present inventors synthesized 15 amino acids at the C-terminus of human beta defensin-3 and identified that such a peptide alone can inhibit the differentiation of osteoclasts, which are bone destruction cells, and inhibit bone destruction in an *in-vivo* animal model. Based on this finding, the present invention has been completed.

### [Disclosure]

### [Technical Problem]

It is one object of the present invention to provide a peptide having excellent bone resorption inhibitory activity.

It is another object of the present invention to provide a pharmaceutical composition and health functional food for preventing or treating bone diseases containing the peptide as an active ingredient.

It is another object of the present invention to provide a method for preventing or treating bone diseases comprising administering the pharmaceutical composition containing the peptide as an active ingredient.

It is another object of the present invention to provide a use of the pharmaceutical composition containing the peptide as an active ingredient for preventing or treating bone diseases.

It is another object of the present invention to provide a use of the pharmaceutical composition containing the peptide as an active ingredient for the manufacturing medicament for preventing or treating bone diseases.

### [Technical Solution]

In accordance with one aspect of the present invention, the above and other objects can be accomplished by the provision of a peptide for inhibiting bone absorption represented by an amino acid sequence of SEQ ID NO: 7.

In accordance with another aspect of the present invention, there is provided a pharmaceutical composition for preventing or treating bone diseases containing, as an active ingredient, a peptide for inhibiting bone absorption represented by an amino acid sequence of SEQ ID NO: 7.

In accordance with another aspect of the present invention, there is provided a method for preventing or treating bone diseases comprising administering a pharmaceutical composition for preventing or treating bone diseases containing, as an active ingredient, a peptide for inhibiting bone absorption represented by an amino acid sequence of SEQ ID NO: 7.

In accordance with another aspect of the present invention, there is provided a use of a pharmaceutical composition for preventing or treating bone diseases containing, as an active ingredient, a peptide for inhibiting bone absorption represented by an amino acid sequence of SEQ ID NO: 7, for preventing or treating bone diseases.

In accordance with another aspect of the present invention, there is provided a use of a pharmaceutical composition for preventing or treating bone diseases containing, as an active ingredient, a peptide for inhibiting bone absorption represented by an amino acid sequence of SEQ ID NO: 7, for manufacturing medicament for preventing or treating bone diseases.

In accordance with another aspect of the present invention, there is provided a health functional food for preventing or alleviating bone diseases containing, as an active ingredient, a peptide for inhibiting bone absorption represented by an amino acid sequence of SEQ ID NO: 7.

### [Description of Drawings]

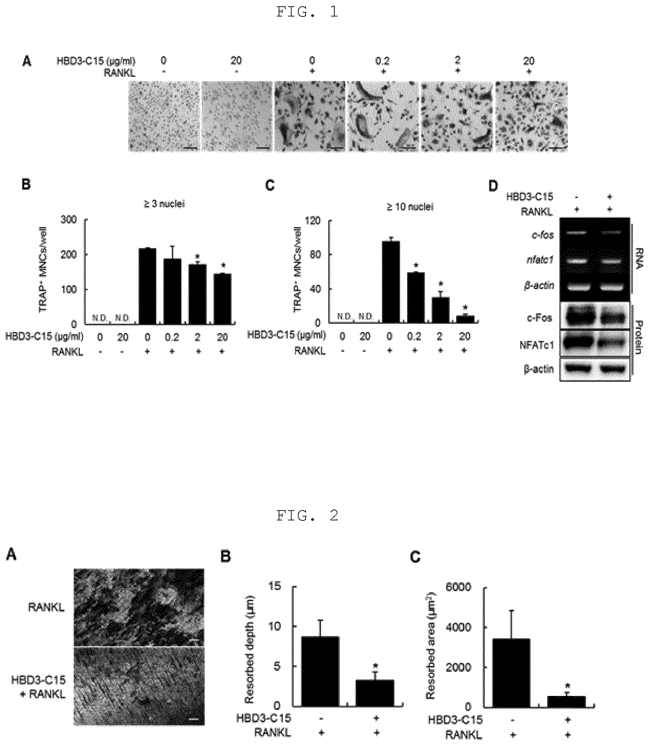

FIG. 1 shows the result of determination of the effect of HBD3-C15 on inhibition of osteoclast differentiation, wherein A shows the result of TRAP staining analysis of mouse-bone-marrow-derived macrophages, B shows the number of cells having 3 or more nuclei, C shows the number of cells having 10 or more nuclei, and D shows the result of determination of expression of c-fos and NFATc1 at the RNA and protein levels.
FIG. 2 shows the result of determination of the effect of HBD3-C15 on inhibition of bone resorption activity of osteoclasts, wherein A is a confocal microscopy image showing a bone resorption depth and a bone resorption area of mouse-bone-marrow-derived macrophages, and B and C show the result of measurement of a bone resorption depth and a bone resorption area using LSM Image Browser software.

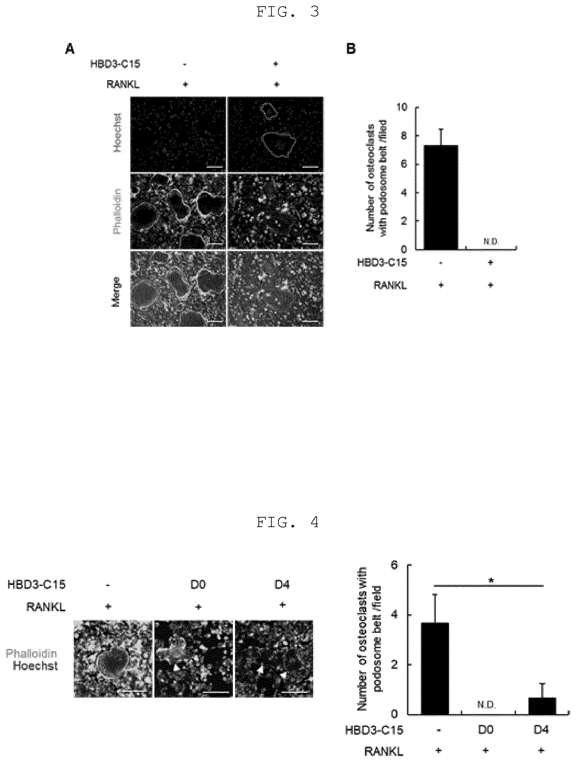

FIG. 3 shows the result of confocal microscopy to determine the effect of HBD3-C15 on inhibition of the formation of a podosome belt of osteoclasts.
FIG. 4 shows the result of confocal microscopy to determine the effect of HBD3-C15 on the destruction of a podosome belt of differentiated mature osteoclasts.

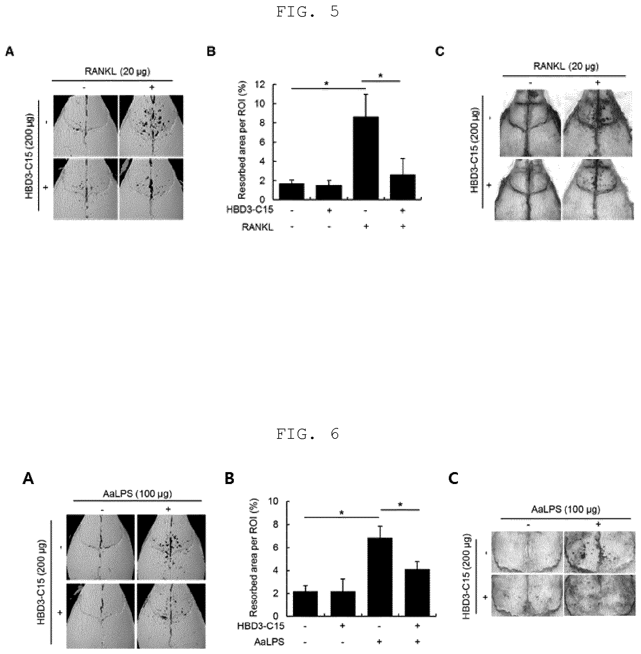

FIG. 5 show the result of determination of the effect of HBD3-C15 on inhibition of bone destruction, wherein A and B show the result of scanning using micro-CT and analysis regarding the level of skull bone fracture in the mouse calvarial implantation model, and C shows the number of osteoclasts of the mouse skull measured using TRAP staining;
FIG. 6 shows the result of determination of the effect of HBD3-C15 on inhibition of bone destruction by in the skull absorbed by lipopolysaccharide of *Aggregatibacter actinomycetemcomitans* (A.a.LPS), which is a bacterium causing aggressive periodontitis, wherein A and B show the result of scanning using micro-CT and analysis regarding the skull, and C shows the number of osteoclasts of the mouse skull measured using TRAP staining.

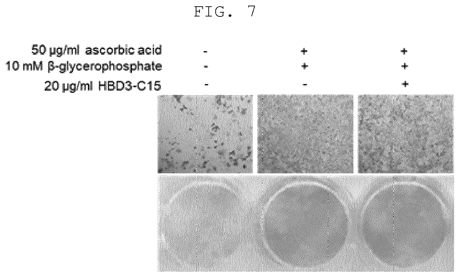

FIG. 7 shows the result of determination of the effect of HBD3-C15 on osteoblast differentiation.

### [Best Mode]

Unless defined otherwise, all technical and scientific terms used herein have the same meanings as appreciated by those skilled in the field to which the present invention pertains. In general, the nomenclature used herein is well-known in the art and is ordinarily used.

In the present invention, a peptide fragment (HBD3-C15; SEQ ID NO: 7) derived from human beta-defensin-3 (HBD3) was synthesized, and the effects of HBD3-C15 on inhibition of osteoclast differentiation and bone resorption were determined. Also, inhibition of activity of differentiated mature osteoclasts having bone destruction ability was determined *in vitro* and *in vivo.* As a result, HBD3-C15 can be sufficiently utilized as a bone resorption inhibitor and can be used as a therapeutic agent for various bone diseases.
SEQ ID NO: 7 (HBD3-C15): G-K-C-S-T-R-G-R-K-C-C-R-R-K-K

Thus, in one aspect, the present invention is directed to a peptide for inhibiting bone absorption represented by the amino acid sequence of SEQ ID NO: 7.

As used herein, the term "peptide" refers to a compound in which not less than 2 and not more than 200 alpha-amino acids are linked by a peptide bond.

The amino acid sequence according to the present invention can be easily modified by addition, deletion, insertion or a combination thereof. Accordingly, peptides or proteins having a high homology with SEQ ID NO: 7, for example, peptides and proteins having a homology of 70% or more, preferably 80% or more therewith, should be also interpreted to fall within the scope of the present invention.

As used herein, the term "homology" is intended to indicate the degree of similarity to the amino acid sequence of a wild-type protein, and includes a sequence having the same sequence as the amino acid sequence of the present invention at the percentage described above. Such homology can be determined by visually comparing the two sequences, but can be determined using bioinformatic algorithms that analyze the degree of homology by aligning the sequences to be compared side by side. The homology between the two amino acid sequences can be expressed as a percentage. Useful automated algorithms are available in the GAP, BESTFIT, FASTA and TFASTA computer software modules of the Wisconsin Genetics Software Package (Genetics Computer Group, Madison, W, USA). Automated alignment algorithms in the module include sequence alignment algorithms of Needleman & Wunsch, Pearson & Lipman, and Smith & Waterman. Determinations of algorithms and homology for other useful alignments are automated in software including FASTP, BLAST, BLAST2, PSIBLAST and CLUSTAL W.

In the present invention, the peptide may be derived from human beta defensin-3 (HBD3).

As used herein, the term "human beta-defensin-3 (HBD3)" refers to a human-derived antibiotic that is expressed in a large amount in the oral cavity, respiratory organs and digestive organs and has an antibiotic effect on bacteria.

In the present invention, the peptide inhibits the activity of mature osteoclasts and activity of the mature osteoclasts may be induced by RANKL (receptor activator of NFκB ligand) or lipopolysaccharide of *Aggregatibacter actinomycetemcomitans* (A.a. LPS).

In the present invention, the term "osteoclast" refers to a cell that performs the functions of destroying and absorbing bone tissue. Osteoclasts are giant cells having a diameter of 20 to 100 µm and respectively containing about 2 to 20 nuclei. Osteoclasts are differentiated from macrophages, and the formation of osteoclasts is induced by a macrophage-colony-stimulating factor (M-CSF), a receptor activator of nuclear factor-kappaB ligand (RANKL) and a co-stimulatory factor of the activators, which mutually cooperate.

In the present invention, the term "bone resorption" refers to a process in which calcium is released from bone tissue, thus making the bone porous and brittle, and in which the bone matrix and bone minerals are simultaneously removed from the bone. Bone resorption is a physiological phenomenon that occurs during bone growth or remodeling, but may also occur due to inflammation or bone metastasis of cancer cells.

The bone destruction inhibitory activity of the present invention means inhibition of osteoclast differentiation or bone resorption.

As used herein, the term "osteoblasts" refers to cells that secrete the bone matrix out of the cells, are spontaneously wrapped in the bone matrix and converted to bone cells. Osteoblasts are differentiated from mesenchymal progenitors,the periosteum is present on the outside of the osteoblast population, and osteoblasts are present on the inner surface of the periosteum even after bone formation, but the number thereof decreases in aged bones.

In another aspect, the present invention is directed to a pharmaceutical composition for preventing or treating bone diseases containing, as an active ingredient, a peptide for inhibiting bone absorption represented by an amino acid sequence of SEQ ID NO: 7.

In the present invention, the bone disease is preferably a bone-resorption-related disease, and is more preferably selected from the group consisting of osteoporosis, periodontal diseases, inflammatory alveolar bone resorption diseases, Paget's disease, rickets, osteomalacia, nephrotic dystrophy of renal failure patients, osteoarthritis, bone metastasis cancer and inflammatory bone resorption diseases, but is not limited thereto.

In the present invention, the composition may inhibit the differentiation of osteoclasts and may inhibit the activity of mature osteoclasts induced by the receptor activator of NFκB ligand (RANKL).

As used herein, the term "prevention" refers to any action causing the suppression or delay of the onset of a disease by administration of a composition.

As used herein, the term "alleviation" or "treatment" refers to any action that improves or positively alters the symptoms of the disease by administration of the composition.

The pharmaceutical composition according to the present invention may contain a pharmaceutically acceptable carrier. The pharmaceutical composition containing the pharmaceutically acceptable carrier may be selected from a variety of oral or parenteral formulations. The pharmaceutical composition of the present invention may be formulated using a commonly used diluent or excipient such as a filler, a thickener, a binder, a wetting agent, a disintegrant or a surfactant. Solid formulations for oral administration may include tablets, powders, granules, capsules and the like. Such a solid formulation is prepared using at least one compound in combination with at least one excipient such as starch, calcium carbonate, sucrose, lactose or gelatin. Liquid formulations for oral administration may include suspensions, oral liquids, emulsions, syrups and the like, and may include, in addition to a commonly used simple diluent such as water or liquid paraffin, various excipients such as wetting agents, sweeteners, aromatics, preservatives and the like. The formulations for parenteral administration include sterile aqueous solutions, non-aqueous solvents, suspensions, emulsions, lyophilizates and suppositories. Useful non-aqueous solvents and suspension solvents include propylene glycol, polyethylene glycol, vegetable oils such as olive oil and injectable esters such as ethyl oleate. Basic ingredients of suppositories include Witepsol, macrogol, Tween 61, cacao butter, laurin butter, glycerogelatin and the like. Suitable pharmaceutically acceptable carriers and preparations are described in detail in Remington's Pharmaceutical Science (17th ed., 1995).

The pharmaceutical composition of the present invention may have any one formulation selected from the group consisting of tablets, pills, powders, granules, capsules, suspensions, oral liquids, emulsions, syrups, sterile aqueous solutions, non-aqueous solvents, suspensions, emulsions, lyophilizates and suppositories.

The route of administration of the pharmaceutical composition according to the present invention may be any general route, so long as it enables the composition to be delivered to a target tissue. The pharmaceutical composition according to the present invention may be administered intraperitoneally, intravenously, intramuscularly, subcutaneously, intradermally, orally, intrapulmonarily, rectally, or intracellularly, either directly or indirectly. For this purpose, the pharmaceutical composition according to the present invention may be administered by any device capable of delivering the active substance to the target cell.

As used herein, the term "administration" refers to an action of supplying a predetermined substance to a patient by any appropriate method, and the composition according to the present invention may be orally or parenterally administered through any general route enabling the composition to be delivered to a target tissue. The pharmaceutical composition may be administered by any device capable of delivering the active substance to the target cell.

The pharmaceutical composition according to the present invention may be administered in a therapeutically effective amount or a pharmaceutically effective amount, and the term "therapeutically effective amount" or "pharmaceutically effective amount" refers to an amount sufficient for treating a disease at a reasonable benefit/risk ratio applicable to all medical treatments, and the effective dosage level may vary depending on a variety of factors including the type of the patient, severity of the disease, age, gender, activity of the drug, sensitivity of the patient to the drug, the administration time, administration route, excretion rate, the treatment period and drugs used concurrently therewith, and other factors well-known in the pharmaceutical field.

For the treatment of bone diseases, the pharmaceutical composition according to the present invention can be administered alone or in combination with surgery, hormonal therapy, drug therapy and methods using biological response modifiers.

In another aspect, the present invention provides a method for preventing or treating bone diseases comprising administering a peptide for inhibiting bone absorption represented by the amino acid sequence of SEQ ID NO: 7, or a pharmaceutical composition containing the peptide.

In another aspect, the present invention provides a use of a peptide for inhibiting bone absorption represented by the amino acid sequence of SEQ ID NO: 7, or a pharmaceutical composition containing the peptide as an active ingredient, for preventing or treating bone diseases.

In another aspect, the present invention provides a use of a peptide for inhibiting bone absorption represented by the amino acid sequence of SEQ ID NO: 7, or a pharmaceutical composition containing the peptide as an active ingredient, for manufacturing medicament for preventing or treating bone diseases.

In another aspect, the present invention provides a method for inhibiting differentiation of osteoclasts using the peptide for inhibiting bone absorption represented by the amino acid sequence of SEQ ID NO: 7.

In another aspect, the present invention provides a health functional food for preventing or alleviating bone diseases containing, as an active ingredient, the peptide for inhibiting bone absorption represented by the amino acid sequence of SEQ ID NO: 7.

In the present invention, the bone disease is preferably a bone-resorption-related disease and is more preferably selected from the group consisting of osteoporosis, periodontal diseases, inflammatory alveolar bone resorption diseases, Paget's disease, rickets, osteomalacia, nephrotic dystrophy of renal failure patients, osteoarthritis, bone metastasis cancer and inflammatory bone resorption diseases, but is not limited thereto.

In the present invention, the peptide inhibits the activity of mature osteoclasts, and the activity of the mature osteoclasts may be induced by RANKL (receptor activator of NFκB ligand) or A.a.LPS.

As used herein, the term "prevention" refers to any action causing the suppression or delay of the onset of a disease by administration of a composition. As used herein, the term "alleviation" refers to any action that improves or positively alters the symptoms of the disease by administration of the composition.

The food may include, but is not limited to, health supplements, health functional foods, functional foods and the like, and includes natural foods, processed foods and general food materials containing the composition for preventing and alleviating bone-resorption-related diseases according to the present invention.

The food composition for preventing and alleviating bone-resorption-related diseases according to the present invention may be used as it is, or may be used in combination with other foods or food compositions, and may be appropriately used in accordance with a conventional method. The amount of the active ingredient that is mixed may be determined suitably according to the purpose (prevention, health or therapeutic treatment) of use. In general, the composition for preventing and alleviating bone-resorption-related diseases according to the present invention is added in an amount of 0.1 to 70 parts by weight, preferably 2 to 50 parts by weight with respect to 100 parts by weight of the raw material of the food or beverage during preparation of the food or beverage. The effective dose of the composition for preventing and alleviating bone-resorption-related diseases may be used according to the effective dose of the pharmaceutical composition. However, in the case of long-term administration for the purpose of health, hygiene or health management, the effective dose may be not more than the range defined above, and the active ingredient may be used in an amount exceeding the range because there is no problem in terms of safety.

There is no particular limitation as to the kind of the food. The food composition for preventing and alleviating bone-resorption-related diseases may be used as a formulation for oral administration such as a tablet, a hard or soft capsule, a liquid and a suspension, and these formulations may be prepared using acceptable conventional carriers, and for example, formulations for oral administration may be prepared using excipients, binders, disintegrants, lubricants, solubilizers, suspending agents, preservatives, thickeners or the like.

Examples of foods to which the composition for preventing and alleviating bone-resorption-related diseases may be added include meat, sausage, bread, chocolate, candy, snacks, confectioneries, pizza, ramen, other types of noodles, gum, dairy products using ice cream, various soups, beverages, teas, drinks, alcoholic beverages, vitamin complexes and the like, but are not limited to these kinds of food.

### Example

Hereinafter, the present invention will be described in more detail with reference to examples. However, it will be obvious to those skilled in the art that these examples are provided only for illustration of the present invention and should not be construed as limiting the scope of the present invention based on subject matter of the present invention.

### Example 1: Effect of HBD3-C15 on inhibition of osteoclast differentiation

### 1-1: Identification of osteoclast differentiation by TRAP staining

In order to evaluate the effect of HBD3-C15, a peptide fragment derived from human beta-defensin-3 (HBD3) on the differentiation of osteoclasts, which are bone destruction cells, mouse-bone-marrow-derived macrophages were used. The bone marrow-derived macrophages that were used were prepared by isolating bone marrow cells from the long bone of mice and culturing the same at 100% humidity and 37°C in an alpha MEM containing 10% fetal bovine serum, 50 unit/ml of penicillin and 50 µg/ml of streptomycin along with 20 ng/ml of M-CSF for 5 days, while continuously feeding 5% carbon dioxide.

Mouse-bone-marrow-derived macrophages were seeded at 20,000 cells/well on a 96-well plate, treated with 100 ng/ml of receptor activator of nuclear factor-kappaB ligand (RANKL) alone, or a combination of 100 ng/ml of RANKL and 0.2, 2, or 20 µg/ml of HBD3-C15, and cultured for 3 days. In order to determine the degree of differentiation of osteoclasts, tartrate-resistant acid phosphatase (TRAP) staining was performed, and cells with three or more nuclei having osteoclast activity were counted and statistically analyzed.

The result showed that the number of TRAP positive cells increased due to RANKL decreased in a concentration-dependent manner due to HBD3-C15 (FIG. 1A), and the number of cells with three or more nuclei having osteoclast activity was statistically significantly decreased due to HBD3-C15 (FIG. 1B). In addition, the number of cells having ten or more nuclei was found to be significantly decreased due to HBD3-C15 (FIG. 1C), which means that HBD3-C15 effectively inhibits the differentiation of osteoclasts.

### 1-2: Identification of osteoclast differentiation by analysis of expression of master differentiation factor

In addition, expression of c-fos and NFATcl, which are well-known key transcription factors for osteoclast differentiation, was determined at protein and RNA levels.

Mouse-bone-marrow-derived macrophages were treated with 20 ng/ml of M-CSF and 100 ng/ml of RANKL, or 20 ng/ml of M-CSF, 100 ng/ml of RANKL and 20 µg/ml of HBD3-C15 for 24 hours, RNA was extracted, and the quantified RNA and random primers were reacted at 70°C for 5 minutes and reacted in the presence of a reverse transcriptase, reverse transcription buffer and dNTPs at 42°C for 1 hour. The result was amplified by 35 cycles using the synthesized cDNA as a template and using c-Fos and NFATc1-specific oligos as primers in a polymerase chain reaction machine. The primers used are shown in Table 1 below.

The result showed that the expression of c-Fos and NFATcl, increased by RANKL, was reduced by HBD3-C15 (FIG. ID).

**[Table 1]**

| | | Primer (5' -> 3') | SEQ. ID. NO.: |
|---|---|---|---|
| c-Fos | Forward | GCCCGACTACGAGGCGTCATCC | 1 |
| | Reverse | TCCGGCACTTGGCTGCAGC | 2 |
| NFATc1 | Forward | TCGGATCGAGGTGCAGCCCA | 3 |
| | Reverse | GGGCTGACCGCTGGGAACAC | 4 |
| β-actin | Forward | GTGGGGCGCCCCAGGCACCA | 5 |
| | Reverse | CTCCTTAATCTCACGCACGATTTC | 6 |

In addition, in order to determine changes of the protein expression of c-Fos and NFATcl, the proteins were extracted from mouse-bone-marrow-derived macrophages, treated with HBD3-C15 for 48 hours, and 40 µg of the protein was transferred to the PVDF membrane through an SDS-PAGE gel. Then, immunoblotting was performed using c-Fos and NFATc1-specific antibodies.

The result showed that HBD3-C15 inhibited an increase in c-Fos and NFATc1 protein expression attributable to RANKL (FIG. 1D). This result indicates that HBD3-C15 inhibits osteoclast differentiation by reducing the expression of c-fos and NFATcl, which are key transcription factors of osteoclast differentiation.

### Example 2: Determination of decrease of bone resorption activity by HBD3-C15 on dentin discs

The osteoclasts were cultured in mouse-bone-marrow-derived macrophages on dentin discs based on the fact that osteoclasts perform all functions thereof in environments enabling bone absorption. Mouse-bone-marrow-derived macrophages were treated with 20 ng/ml of M-CSF and 100 ng/ml of RANKL, or with 20 ng/ml of M-CSF, 100 ng/ml of RANKL and 20 µg/ml of HBD3-C15, and then cultured for 6 days. The bone resorption area and bone resorption depth were imaged by confocal microscopy and total bone resorption depth and bone resorption area were measured by LSM Image Browser software.

The increase in bone resorption depth and bone resorption area attributable to RANKL was found to be significantly reduced by HBD3-C15 (FIG. 2). This result suggests that HBD3-C15 not only reduces osteoclast differentiation but also decreases actual bone resorption activity.

### Example 3: Inhibition of podosome belt formation in osteoclasts by HBD3-C15

Podosome belt formation of osteoclasts is known to be positively correlated with bone resorption, and bone resorption does not occur well in mice having no podosome belt formation (Matsumoto T. et al., J. Bone Miner. Res. 28:1191-1202, 2013; Vives V. et al., Nat. Commun. 6:6218, 2015).

Thus, in order to determine whether or not the reduction of osteoclast differentiation by HBD3-C15 is affected by podosome belt formation in osteoclasts, mouse-derived bone marrow macrophages were differentiated on a cover glass. Mouse-bone-marrow-derived macrophages were treated with 20 ng/ml of M-CSF and 100 ng/ml of RANKL, or 20 ng/ml of M-CSF, 100 ng/ml of RANKL and 20 µg/ml of HBD3-C15, and cultured for 5 days. The cells were fixed with 4% paraformaldehyde, stained with Alexa-Fluor-488-conjugated-phalloidin, and washed and stained with Hoechst 33258. The degree of staining of cells was observed by confocal microscopy.

As a result, no clear podosome belt (green) formed by RANKL was observed in cells treated with HBD3-C15 (FIG. 3). This result suggests that the decrease in bone resorption of osteoclasts by HBD3-C15 is caused by the absence of podosome belt formation.

### Example 4: Determination of destruction of podosome belt of mature osteoclasts by HBD3-C15

Diseases such as osteoporosis are caused by excessive osteoclast activity, and inhibition of the function of mature osteoclasts having bone destruction ability can maximize the efficacy as a therapeutic agent.

Thus, in order to determine whether or not HBD3-C15 can inhibit the activity of differentiated mature osteoclasts, mouse-bone-marrow-derived macrophages were treated with 20 ng/ml of M-CSF and 100 ng/ml of RANKL on a cover glass for 4 days to induce differentiation of osteoclasts. The osteoclasts were treated with 20 µg/ml of HBD3-C15 on day 4 of differentiation and cultured for 1 day. The cells were fixed with 4% paraformaldehyde, stained with Alexa-Fluor-488-conjugated phalloidin, washed and nuclei-stained with Hoechst 33258. The degree of staining of cells was observed by confocal microscopy.

As a result, a clear podosome belt (green) was formed by RANKL, but an incomplete podosome belt was observed when treating cells with HBD3-C15 on day 4 of differentiation (FIG. 4). This result suggests that HBD3-C15 can inhibit the function of mature osteoclasts that are completely differentiated and then enable bone destruction.

### Example 5: Determination of ability of HBD3-C15 to inhibit bone resorption in vivo

### 5-1: HBD3-C15 inhibiting bone resorption by RANKL

In order to determine the inhibition of bone destruction by HBD3-C15 *in vivo,* a calvarial implantation model, a typical bone loss model, was used.

20 µg of RANKL, a key cytokine that can destroy bones, was soaked in a collagen sheet and was transplanted alone into mouse skulls, or 20 µg of RANKL and 200 µg of HBD3-C15 were soaked in a collagen sheet and then transplanted into mouse skulls. The mice were sacrificed on day 7 of transplantation, and the degree of skull bone destruction was analyzed by scanning via micro-CT.

The result showed that the skull absorbed by RANKL was significantly reduced by HBD3-C15 (FIGS. 5A and 5B) .

After micro-CT analysis, TRAP staining was performed to determine the number of osteoclasts in the skull. The result showed that the number of osteoclasts increased in the skulls of mice transplanted with RANKL alone was significantly reduced when HBD3C-15 was transplanted into mice (FIG. 5C).

### 5-2: HBD3-C15 inhibiting bone resorption by A.a.LPS

In addition, lipopolysaccharide of *Aggregatibacter actinomycetemcomitans* (A.a.LPS), a bacterium known to cause aggressive periodontitis accompanied with rapid alveolar bone loss, induced resorption of mouse skulls, and whether such resorption can be inhibited by HBD3-C15 was determined

The result showed that skull resorption by A.a.LPS was significantly reduced by the transplantation of HBD3-C15, and the number of osteoclasts was also decreased (FIG. 6). Overall, these results suggest that HBD3C-15 is sufficiently useful as a bone destruction inhibitor and HBD3C-15 is useful as a therapeutic or adjuvant agent for various bone diseases caused by differentiation and excessively increased activity of osteoclasts.

### Example 6: Effect of HBD3-C15 on osteoblast differentiation

In order to evaluate the effect of HBD3-C15 on differentiation of osteoblasts, which are bone formation cells, the osteoblast cell line MC3T3-E1 (Subclone 4) was used. The cells were prepared by culturing in alpha-MEM medium without ascorbic acid containing 10% fetal bovine serum, 50 unit/ml of penicillin, and 50 µg/ml of streptomycin at 37°C and 100% humidity while continuously supplying 5% carbon dioxide.

MC3T3-E1 cells were seeded at a density of 25,000 cells/well into a 48-well plate, then treated with a differentiation medium alone supplemented with 50 µg/ml of ascorbic acid and 10 mM of β-glycerophosphate, or in combination with 20 µg/ml of HBD3-C15, and cultured for 6 days. Alkaline phosphate staining was performed to determine the differentiation of osteoblasts and images were obtained through a camera mounted on a microscope.

The result showed that the differentiation of osteoblasts was increased by the use of the differentiation medium supplemented with 50 µg/ml of ascorbic acid and 10 mM β-glycerophosphate, and there was no difference in the degree of differentiation even in cells treated in combination with HBD3-C15 (FIG. 7). The result indicate that HBD3-C15 does not affect the differentiation of osteoblasts.

### [Industrial Applicability]

The peptide for inhibiting bone resorption according to the present invention is capable of inhibiting bone destruction by inhibiting the differentiation and activity of osteoclasts and thus can be used as a therapeutic agent for various bone diseases caused by an excessive increase in the activity of osteoclasts.

Although specific configurations of the present invention have been described in detail, those skilled in the art will appreciate that this description is provided to set forth preferred embodiments for illustrative purposes and should not be construed as limiting the scope of the present invention. Therefore, the substantial scope of the present invention is defined by the accompanying claims and equivalents thereto.

### [Sequence Listing Free Text]

An electronic file is attached.

## Claims

1. A peptide for inhibiting bone absorption represented by an amino acid sequence of SEQ ID NO: 7.

2. The peptide according to claim 1, wherein the peptide is derived from human beta-defensin-3 (HBD3).

3. The peptide according to claim 1, wherein the peptide inhibits differentiation of osteoclasts.

4. The peptide according to claim 1, wherein the peptide inhibits activity of mature osteoclasts.

5. A pharmaceutical composition for preventing or treating bone disease comprising, as an active ingredient, the peptide for inhibiting bone absorption according to claim 1.

6. The pharmaceutical composition according to claim 5, wherein the bone disease is selected from the group consisting of osteoporosis, periodontal diseases, inflammatory alveolar bone resorption diseases, Paget's disease, rickets, osteomalacia, nephrotic dystrophy of renal failure patients, osteoarthritis, bone metastasis cancer and inflammatory bone resorption diseases.

7. The pharmaceutical composition according to claim 5, wherein the pharmaceutical composition inhibits differentiation of osteoclasts.

8. The pharmaceutical composition according to claim 5, wherein the pharmaceutical composition inhibits activity of mature osteoclasts.

9. A health functional food for preventing or alleviating bone disease comprising, as an active ingredient, the peptide for inhibiting bone absorption according to claim 1.

10. The health functional food according to claim 9, wherein the bone disease is selected from the group consisting of osteoporosis, periodontal diseases, inflammatory alveolar bone resorption diseases, Paget's disease, rickets, osteomalacia, nephrotic dystrophy of renal failure patients, osteoarthritis, bone metastasis cancer and inflammatory bone resorption diseases.

11. The health functional food according to claim 9, wherein the health functional food inhibits differentiation of osteoclasts.

12. The health functional food according to claim 9, wherein the health functional food inhibits activity of mature osteoclasts.
